# DEMANDE DE BREVET EUROPEEN

(11) **EP 2 638 928 A1**
(43) Date de publication de la demande: **18.09.2013**
(21) Numéro de dépôt: 12159818.9
(22) Date de dépôt: 16.03.2012
(51) Int. Cl.: A61N 1/16

(54) **Magnéto-culture**

(71) Demandeur: NTN Capital Partners, 8952 Schlieren (CH)
(72) Inventeur: Grolimund, Eduard, 8952 Schlieren (CH)

(57) **Abrégé**

PROCÉDÉ DE GESTION ET PRODUCTION DE CHAMPS ENERGÉTIQUES COSMOS /TELLURIQUE TOURBILLONANTS CONCENTRÉS DÉNOMMÉS « VORTEX » DESTINÉ À CRÉER DES MODIFICATIONS, DES PROTECTIONS ET/OU DES DYNAMISATIONS ENERGÉTIQUES D'UN OU PLUSIEURS LIEUX GÉOGRAPHIQUES, ET DISPOSITIF LE METTANT EN OEUVRE.

## Description

La présente invention concerne un procédé de production de champs énergétiques cosmos/telluriques tourbillonnants dénommés « Vortex », destiné à créer des modifications, des protections et/ou des dynamisations énergétiques d'un ou plusieurs lieux géographiques.

La surface géographique de notre planète est constituée d'une multitude de réseaux « énergétiques naturels » sous forme de maillage ou quadrillage. Ces quadrillages ou maillages sont formés de lignes énergétiques, elles-mêmes composées d'ondes porteuses et d'ondes portées. Ces réseaux sont spécifiquement appelés réseaux Hartmann ou réseaux Curie pour ne citer que les plus connus d'entre eux.

L'énergie informationnelle de ces réseaux a des incidences fondamentales sur tous les organismes vivants, êtres humains, animaux, plantes, y compris l'eau et les minéraux. En effet, ces courants énergétiques informationnels peuvent-être selon le cas, bon ou néfaste pour les processus de vie en générale.

Le fait de connaître leurs existence, de savoir les détecter, connaitre leurs fonctionnements spécifiques, de pouvoir agir activement sur leurs paramètres intrinsèques, nous permet maintenant d'effectuer des réglages capables d'optimiser les qualités dynamiques, énergétiques de lieux géographiquement perturbés. Après ré harmonisations et rééquilibrages énergétiques de ces lieux, les personnes présentes sur ces sites seront dès lors dans un climat de « bien être ».

Pour aboutir à ces résultats, il a été mis au point une technologie spécifique se déclinant en deux sections principales d'applications, appelées respectivement : « Magnéto Protection ou Protection Géomagnétique » et, ; « Magnéto Culture ».

La Magnéto Culture issue de cette technologie est orientée spécifiquement ver le domaine agronomique pour l'optimisation des croissances du domaine végétales, le dispositif consiste à une gestion active des lignes de champs énergétiques naturelles circulant autour de la terre et, par la mise en place précise de ces condensateurs dits de « Magnéto Culture » sur des emplacements spécifiques.

Ces lignes énergétiques sont inscrites sur plans et notées après détection à l'aide d'un équipement spécial de mesure électronique approprié.

Cela peut-être également réalisé par la méthode du Pr. Dr. Walter Kunnen à l'aide d'une antenne de Lecher étalonnée, et utilisée par un radiesthésiste scientifique confirmé pour l'exactitude de ses mesures.

Le nombre et le positionnement de base pour réaliser ce circuit énergétique est de l'ordre de 7 condensateurs disposés en hexagone de façon équidistante.

Ces 7 condensateurs sont disposés sur ou dans le sol. Il faut une profondeur d'environ 30 à 40 cm par condensateur, s'ils sont immergés dans le sol.

Il y a un condensateur au centre, puis 6 autres sur la périphérie hexagonale.

Dès le moment ou les condensateurs de « Magnéto Culture » sont disposés correctement sur leur lieu d'action et dès la mise en place du dernier sur la périphérie, le système se met automatiquement en action par la création de champs énergétiques tourbillonnants, à la manière du cyclone dont la rotation est dirigée dans le sens anti horaire, (sens lévogyre). Il est invisible à l'oeil nu, mais très actif dans le temps et sur les éléments physiques se tenant dans sa périphérie d'action, dont le diamètre moyen avoisine les 60 mètres.

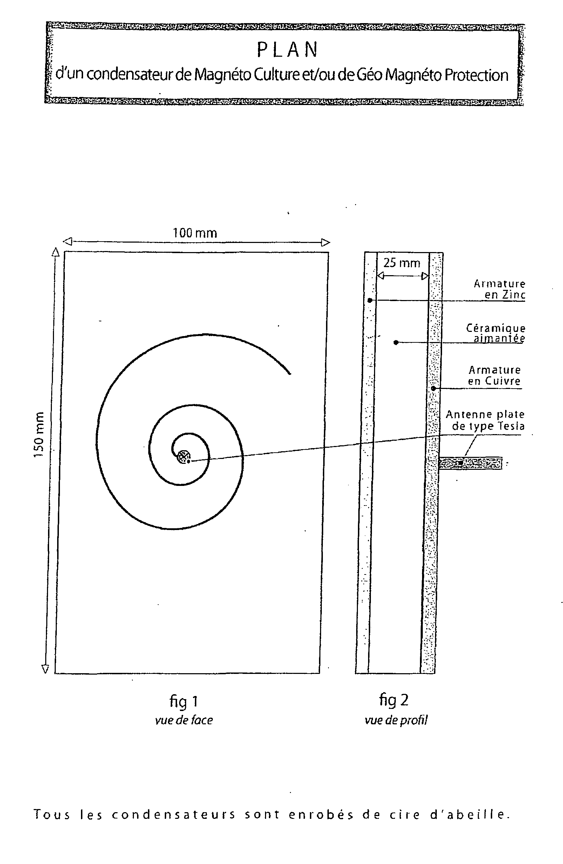

## Revendications

1. Procédé pour la gestion et la production de champs énergétiques Cosmos/Telluriques concentrés, tourbillonnants, destinés à créer des modifications, des protections et/ou des dynamisations énergétiques d'un ou plusieurs lieux géographiques.

2. Le procédé de base requiert un minimum de sept condensateurs mis en connexion magnétique les uns aux autres, créant ainsi un circuit magnétique à partir d'un centre défini en rapport d'une structure hexagonale servant de guide et de locomotive pour produire une concentration des flux Cosmos/Telluriques sur une surface donnée, ainsi qu'une mise en rotation de ces flux à la manière d'un cyclone atmosphérique.

3. Que ces résultats se caractérisent par des changements de niveaux énergétiques des lieux traités selon les réglages définis, ainsi que par la filtration et le contrôle des ondes portées en rapport des condensateurs utilisés par les ondes porteuses filtrées par ces mêmes condensateurs.

4. Que l'accordage en fréquence des condensateurs accordés sur les fréquences naturelles de l'eau saine, et par conséquent en résonnances commutées avec les grands réseaux Hartmann et Curie, dont les emplacements détectés avec précisions pour leurs particularités spécifiques en les utilisant comme amplificateur naturel de signaux biologiques.

5. Procédé conforme à la revendication 1, **se caractérisant par le fait que** la propagation de signaux biologiques, ainsi que les signaux Cosmos/Telluriques s'effectuent dans le faisceau énergétique tourbillonnant créé par les « Vortex » magnétiques, et engendrés par les dispositions et l'orientation des condensateurs dans l'espace, et en accord de phase face à l'orientation et aux flux magnétiques naturels.

6. Procédé pour les traitements énergétiques des lieux par la mise en oeuvre d'un procédé conforme à la revendication 1&2 **caractérisé par le fait que** ce système n'utilise en aucun cas des énergies de bases produites par l'homme, mais uniquement par des flux énergétiques naturels d'origine Cosmos/Telluriques.

7. Procédé capable de rééquilibrer l'énergétique environnemental de l'habitat, d'une zone géographique ou d'un lieu, et par conséquent de créer indirectement une protection contre les perturbations d'ordre électromagnétiques envers l'être humain et le vivant en général.

8. Que le procédé conforme à la revendication 1&2 mis correctement en place et bien réglé est très efficace dans l'agriculture pour son effet d'amplification sur la croissance des plantes, tout en réduisant progressivement l'apport de produits chimiques traditionnels.

9. ° le dispositif est composé d'une céramique anisotropique, d'une plaque de cuivre , d'une plaque de zinc ,d'une antenne plate de type « tesla », l'ensemble enrobé d'un diélectrique en cire d'abeille, et le tout confiné dans une coque de protection en géo polymère. Ce procédé mis en place ne nécessite aucune modification technique et rajout d'autres équipements pour son bon fonctionnement.

## Revendications modifiées

### Revendications modifiées conformément à la règle 137(2) CBE.

**1.** Le procédé de base requiert un minimum de sept condensateurs mis en connexion magnétique les uns aux autres, créant ainsi un circuit magnétique à partir d'un centre défini en rapport d'une structure hexagonale servant de guide et de locomotive pour produire une concentration des flux Cosmos/Telluriques sur une surface donnée, ainsi qu'une mise en rotation de ces flux à la manière d'un cyclone atmosphérique.

**2.** Que ces résultats se caractérisent par des changements de niveaux énergétiques des lieux traités selon les réglages définis, ainsi que par la filtration et le contrôle des ondes portées en rapport des condensateurs utilisés par les ondes porteuses filtrées par ces mêmes condensateurs.

**3.** Que l'accordage en fréquence des condensateurs accordés sur les fréquences naturelles de l'eau saine, et par conséquent en résonnances commutées avec les grands réseaux Hartmann et Curie, dont les emplacements détectés avec précisions pour leurs particularités spécifiques en les utilisant comme amplificateur naturel de signaux biologiques.

**4.** Procédé conforme à la revendication 1, **se caractérisant par le fait que** la propagation de signaux biologiques, ainsi que les signaux Cosmos/Telluriques s'effectuent dans le faisceau énergétique tourbillonnant créé par les « Vortex » magnétiques, et engendrés par les dispositions et l'orientation des condensateurs dans l'espace, et en accord de phase face à l'orientation et aux flux magnétiques naturels.

**5.** Procédé pour les traitements énergétiques des lieux par la mise en oeuvre d'un procédé conforme à la revendication 1&2 **caractérisé par le fait que** ce système n'utilise en aucun cas des énergies de bases produites par l'homme, mais uniquement par des flux énergétiques naturels d'origine Cosmos/Telluriques.

**6.** Procédé capable de rééquilibrer l'énergétique environnemental de l'habitat, d'une zone géographique ou d'un lieu, et par conséquent de créer indirectement une protection contre les perturbations d'ordre électromagnétiques envers l'être humain et le vivant en général.

**7.** Que le procédé conforme à la revendication 1&2 mis correctement en place et bien réglé est très efficace dans l'agriculture pour son effet d'amplification sur la croissance des plantes, tout en réduisant progressivement l'apport de produits chimiques traditionnels.

**8.** le dispositif est composé d'une céramique anisotropique, d'une plaque de cuivre , d'une plaque de zinc ,d'une antenne plate de type « tesla », l'ensemble enrobé d'un diélectrique en cire d'abeille, et le tout confiné dans une coque de protection en géo polymère. Ce procédé mis en place ne nécessite aucune modification technique et rajout d'autres équipements pour son bon
fonctionnement.
